# EUROPEAN PATENT SPECIFICATION

(11) **EP 0 437 291 B1**
(45) Date of publication and mention of the grant of the patent: **22.12.1993**
(21) Application number: 91200021.3
(22) Date of filing: 08.01.1991
(51) Int. Cl.: A61M 25/00, B29C 45/14

(54) **Method for manufacturing a soft tip catheter**
Verfahren zur Herstellung eines Katheters mit weicher Spitze
Procédé de fabrication d'un cathéter à embout mou

(30) Priority: 09.01.1990 NL 9000054
(43) Date of publication of application: 17.07.1991
(73) Proprietor: CORDIS EUROPA N.V., NL-9301 LJ Roden (NL)
(72) Inventor: Wijkamp, Arnoldus Cornelius Johannes Maria, NL-9302 AW Roden (NL); Antoni, Robert, NL-9725 CD Groningen (NL); Griep, Wilhelmus Antonius Maria, NL-9301 PK Roden (NL)
(74) Representative: 't Jong, Bastiaan Jacobus

(56) References cited:
- EP-A- 0 303 487
- FR-A- 2 550 454
- US-A- 4 210 478

## Description

The invention relates to a method for manufacturing a catheter as described in the introductory part of claim 1.

Such a method is known from EP-A O 303 487. This publication relates to the manufacturing of a soft tip catheter. The body of a catheter is prepared in a specific way before the tip is injection moulded thereon.

The object of the invention is to provide a more efficient manufacturing method.

With the method according to the invention, this is achieved with the measures of the characterizing part of claim 1. When the end portion is injected in liquid form at least a portion of the heated liquid plastic moves against the end surface of the basic body, this end surface is well heated and the material thereof at the location of the end surface will become fused with the material of the end portion. When the basic body is manufactured from a thermoplastic plastic, the material thereof at the location of the end surface can like-wise melt as a result of the flow of heated plastic and mix with this flow, so that a very good join results.

According to an important aspect of the invention the end portion can be formed from a plastic that is soft relative to the material of the basic body. A catheter is hereby manufactured whereof the end which must be inserted into a patient during use thereof is soft, so that the risk of internal injury is to a large degree excluded.

With the method according to the invention an end portion can be formed which is comparatively long relative to the diameter, for instance for catheters for angiographic purposes, which comprise a basic body with a sheath of braided metal wire and a relatively flexible end portion curved into a determined shape. The method according to the invention can also be used for only arranging a comparatively short, atraumatic soft end portion. In that case the basic body may already comprise an end portion injected in liquid form or otherwise arranged, whereby further arranged with the method according to the invention is another additional end portion of soft plastic with a length of at most several times the diameter thereof.

The invention also relates to and provides a device for manufacturing a catheter as defined in claim 3

From US-A-4 210 478 a device is known for injection moulding of an end portion to a catheter basic body, comprising a die with a recess for taking up the basic body and having a mould cavity defining the end portion of the catheter. In this known device however, the feeding channel for feeding heated, molten plastic under pressure to the mould cavity debauches, in view of the recess for taking up the catheter basic body, on the opposite end of the mould.

The invention is further elucidated in the following description of several embodiments.

Fig. 1 shows a perspective view of a catheter as can be manufactured with the invention.

Fig.2 shows a partial lengthwise section of a device according to the invention.

Fig. 3 shows a section corresponding with fig. 2 of a die according to another embodiment.

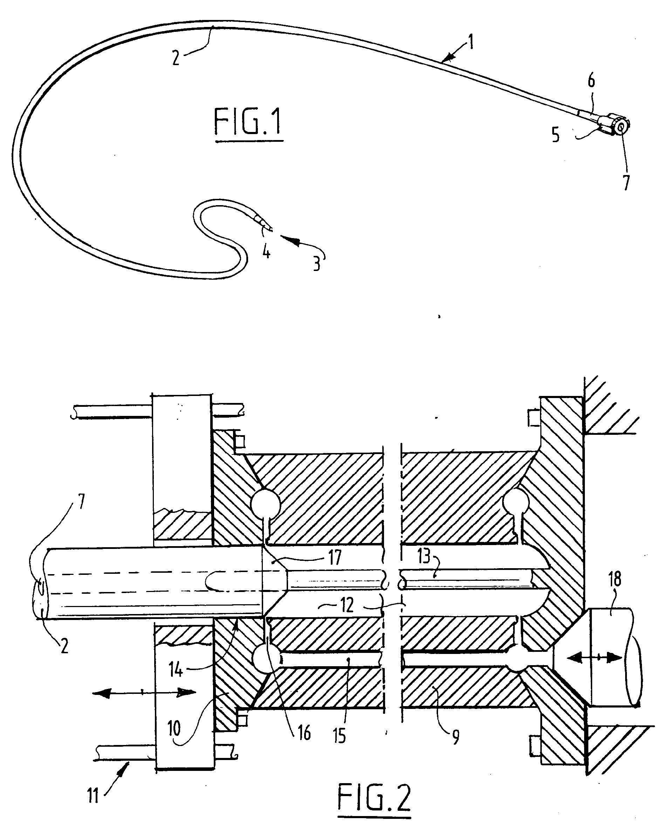

Fig. 1 shows a catheter 1 manufactured using the method and device according to the invention. This catheter 1 comprises in further per se known manner a basic body 2, which may for instance contain an embedded sheath of braided steel wire, having on a connecting end thereof a connecting member 5 arranged together with a reinforcement or a strain relief 6. Arranged on the other end of the basic body 2 is an end portion 3 which has two different material properties relative to the base material. The catheter 1 shown is particularly a catheter for angiographic purposes, wherein the end portion 3 is relatively flexible. In the catheter 1 shown, the extremity of the end portion 3 is provided with a soft point 4. This soft point 4 and/or the remaining portion of the end portion 3 are formed by injection moulding and injected in liquid form onto the basic body 2.

The device 8 shown schematically in fig. 2 is an embodiment of a device with which a catheter of the sort shown in fig. 1 can be manufactured. The device 8 comprises a fixedly mounted die part 9 and a moveable die part 10 arranged for movement relative to this fixed die part 9 by schematically designated guidance means 11. Recessed into the fixed die part 9 is a mould cavity 12 with the shape of the end portion for manufacture. Connecting axially to the mould cavity 12 in the moveable die part 10 is a recess 14, into which an end of the basic body 2 can be arranged. A mandrel 13 extends from the end of the mould cavity 12 lying opposite the recess 14 into the recess 14. In the closed position of the die 9, 10 shown in fig. 2 this mandrel 13 protrudes into the central channel 7 in the basic body 2 and defines an elongation of the central channel 7 in the end portion to be formed.

A feed channel designated in its entirety by 15 is formed in the die 9 for feeding heated, molten plastic under pressure to the mould cavity 12. This plastic is fed via the die 18 of an injection moulding machine which is otherwise conventional and therefore does not require further description here. A portion 16 of the channel 15 debouches close to the recess 14, so that liquid heated plastic flowing from this feed channel portion 16 moves against the connecting end surface 17 of the basic body 2. The material in this connecting end surface 17 is hereby well heated and may even be melted, so that a very good adhesion occurs of the material of the end portion to the material of the basic body 2.

Although the die shown in fig. 2 is a die in which the parts 9, 10 can be moved apart from one another in the axial direction, a die for manufacturing relatively long end portions will preferably be divisible along a plane through the heart line. The mandrel 13 can be detachable in order to facilitate demoulding. A person skilled in the art of injection moulding can, by simple application of his professional knowledge, design the most suitable die construction.

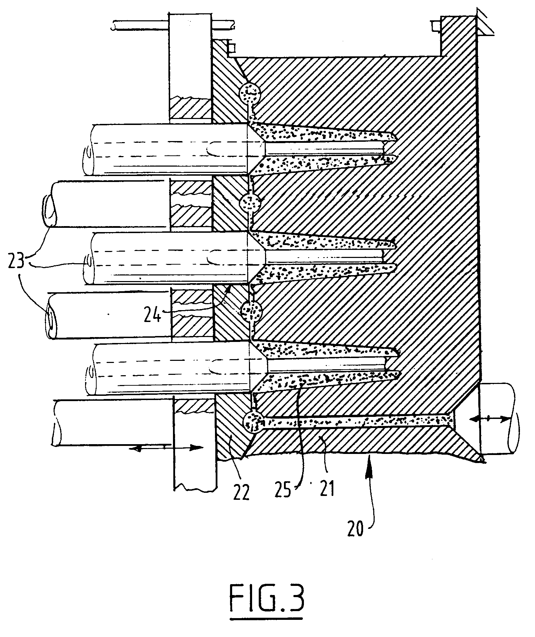

The device from fig. 3 is intended for injecting in liquid form an end portion the length of which amounts at most to only several times the diameter. This end portion in particular is manufactured from a soft plastic, so that the final catheter has an atraumatic point. The die 20 shown here corresponds to a considerable extent with the device 8 from fig. 2, except that the die 20 is embodied as a multiple die in which various basic bodies 23 can be provided with an end portion 25 injected in liquid form with one injection moulding process. The die 20 comprises die parts 21 and 22 moveable relative to each other, wherein in the one die part the mould cavities for forming the end portions 25 are recessed, and the other die part 22 has the recesses 24 for arranging therein of the ends of the basic body 23. These ends to be arranged in the recesses 24 can themselves also be parts already injected in liquid form on a sub-basic body.

The catheter 1 shown in fig. 1 can thus be manufactured such that in a first injection moulding process an end portion 3 of flexible plastic is injected in liquid form on the basic body 2 having an embedded sheath of braided metal wire, whereafter in a following injection moulding process a soft point 4 is formed on the end portion injected in liquid form.

## Claims

1. Method for manufacturing a catheter, comprising manufacturing a tube-like basic body (2) with a central channel, arranging a connecting member (5) on a connecting end thereof, forming a tube-like end portion (3) with material properties different from said basic body (2) on the other end of said basic body (2), by injection moulding, said end portion (3) during forming being injected in liquid form directly onto said basic body (2), **characterized in** **that** during injection in liquid form of the end portion (3) onto the basic body (2), a flow of heated, liquid plastic for forming the end portion (3) is supplied in a position adjacent the connecting end surface and against this surface.

2. Method as claimed in claim 1, **characterized in** **that** the end portion (3) is formed from a plastic that is soft relative to the material of the basic body (2).

3. Device for manufacturing a catheter, comprising an injection moulding device with a die (9, 10) which has a mould cavity (12) defining an end portion of the catheter, a feed channel (15) leading to said mould cavity (12) for feeding heated, molten plastic under pressure to said mould cavity (12), wherein connected to said mould cavity is a recess (14) for enclosed arranging of an end of a basic body (2) for a catheter and wherein a mandrel (13) extends from the end of said mould cavity (12) lying opposite said recess (14), through said mould cavity into said recess, which mandrel defines an axial channel of said end portion, **characterized in that** at least a portion (16) of the feed channel debouches close to the recess (14) such that liquid heated plastic flowing out of this feed channel (15) is directed against the end surface (17) of a catheter basic body (2) arranged in said recess (14).

## Patentansprüche

1. Verfahren zur Herstellung eines Katheders, bei welchem ein schlauchartiger Grundkörper (2) mit einem Mittelkanal hergestellt und ein Verbindungsglied (5) an einem Verbindungsende angebracht wird und ein Hülsenrendabschnitt (3) mit Materialeigenschaften, die unterschiedlich sind von jenen des Grundkörpers (2) an dem anderen Ende des Grundkörpers durch ein Spritzgußverfahren angeformt wird, wobei dieser Endabschnitt (3) während der Formgebung in flüssiger Form direkt auf den Grundkörper (2) aufspritzt wird,
dadurch gekennzeichnet, daß während der Endabschnitt (3) in flüssiger Form auf den Grundkörper (2) aufgespritzt wird, eine Strömung erhitzten flüssigen Plastikmaterials zur Erzeugung des Endabschnitts (3) nach einer Stelle benachbart zur Verbindungsstirnfläche und gegen diese Stirnfläche zugeführt wird.

2. Verfahren nach Anspruch 1,
dadurch gekennzeichnet, daß der Endabschnitt (3) aus einem Plastikmaterial hergestellt wird, welches weich ist relativ zu dem Material des Grundkörpers (2).

3. Vorrichtung zur Herstellung eines Katheders mit einer Spritzgußvorrichtung, die eine Form (9,10) aufweist, die einen Formhohlraum (12) besitzt, der einen Endabschnitt des Katheders definiert, mit einem Zuführungskanal (15), der nach dem Formhohlraum (12) führt, um erhitztes geschmolzenes Plastikmaterial unter Druck dem Hohlraum (12) zuzuführen, wobei mit dem Formhohlraum eine Ausnehmung (14) verbunden ist, um ein Ende eines Basiskörpers (2) für ein Katheder zu umschließen und wobei ein Kern (13) vom jenem Ende des Formhohlraumes (12) das der Ausnehmung (14) entgegengesetzt ist, durch den Formhohlraum in die Ausnehmung einsteht, wobei dieser Kern einen Axialkanal des Endabschnitts definiert,
dadurch gekennzeichnet, daß wenigstens ein Teil (16) des Zuführungskanals dicht benachbart zu der Ausnehmung (14) durchbricht, derart, daß flüssiges erhitztes Plastikmaterial, das aus diesem Zuführungskanal (15) ausfließt, gegen die Stirnfläche (17) des Kathedergrundkörpers (2) gerichtet wird, der in dieser Ausnehmung (14) liegt.

## Revendications

1. Procédé pour fabriquer un cathéter, consistant à fabriquer un corps de base de type tube (2) avec un canal central, à prévoir un organe de raccordement (5) sur une extrémité de raccordement de ce dernier, à former une partie d'extrémité de type tube (3) avec des propriétés de matériau différentes dudit corps de base (2) sur l'autre extrémité dudit corps de base (2), par moulage par injection, ladite partie d'extrémité (3), pendant le formage, étant injectée sous forme liquide directement sur ledit corps de base (2), **caractérisé en ce que** pendant l'injection sous forme liquide de la partie d'extrémité (3) sur le corps de base (2), un écoulement de plastique liquide et chauffé pour former la partie d'extrémité (3) est fourni dans une position adjacente à la surface d'extrémité de raccordement et contre cette surface.

2. Procédé selon la revendication 1, **caractérisé en** **ce que** la partie d'extrémité (3) est formée à partir d'un plastique qui est souple par rapport au matériau du corps de base (2).

3. Dispositif pour fabriquer un cathéter, comprenant un dispositif de moulage par injection avec une matrice (9, 10) qui comporte une cavité formant moule (12) définissant une partie d'extrémité du cathéter, un canal d'alimentation (15) conduisant à ladite cavité formant moule (12) pour fournir du plastique, chauffé et fondu sous pression, à ladite cavité formant moule (12), dans lequel un évidement (14) raccordé à ladite cavité formant moule est prévu pour recevoir une extrémité d'un corps de base (2) pour un cathéter et dans lequel un mandrin (13) s'étend à partir de l'extrémité de ladite cavité formant moule (12) située à l'opposé dudit évidement (14), à travers ladite cavité formant moule dans ledit évidement, lequel mandrin définit un canal axial de ladite partie d'extrémité, **caractérisé** **en ce qu'**au moins une partie (16) du canal d'alimentation débouche à proximité de l'évidement (14) de telle sorte que le plastique chauffé et liquide s'écoulant hors de ce canal d'alimentation (15) est dirigé contre la surface d'extrémité (17) d'un corps de base du cathéter (2) prévu dans ledit évidement (14).
